## Europäisches Patentamt

## European Patent Office

## Office européen des brevets

(11) Veröffentlichungsnummer: **0 059 431**
**B1**

(12) **EUROPÄISCHE PATENTSCHRIFT**

(45) Veröffentlichungstag der Patentschrift:
**21.09.83**

(51) Int. Cl.³: **C 07 D 303/48,** C 07 D 301/32

(21) Anmeldenummer: **82101435.4**

(22) Anmeldetag: **25.02.82**

(54) **Verfahren zur Herstellung von reinem Hexafluorpropylenoxid.**

(30) Priorität: **03.03.81 DE 3107967**

(43) Veröffentlichungstag der Anmeldung:
**08.09.82 Patentblatt 82/36**

(45) Bekanntmachung des Hinweises auf die Patenterteilung:
**21.09.83 Patentblatt 83/38**

(84) Benannte Vertragsstaaten:
**BE DE FR GB IT NL**

(56) Entgegenhaltungen:
**DE-A-2 752 998**
**DE-B-1 251 300**

(73) Patentinhaber: **HOECHST AKTIENGESELLSCHAFT,**
**Postfach 80 03 20, D-6230 Frankfurt am Main 80 (DE)**

(72) Erfinder: **Sulzbach, Reinhard Albert, Dr.,**
**Kammererstrasse 10, D-8263 Burghausen/Salzach (DE)**

### Verfahren zur Herstellung von reinem Hexafluorpropylenoxid

Die Erfindung betrifft ein Verfahren zur Reindarstellung von Hexafluorpropylenoxid (HFPO) aus Gemischen, bestehend aus HFPO und Hexafluorpropylen (HFP), durch extraktive Destillation in Gegenwart eines Extraktivmediums.

HFPO wird durch Oxidation von HFP hergestellt, wofür eine Reihe von Verfahren bekannt sind, die sich verschiedenartiger Oxidationsmittel bedienen. Allen Verfahren ist gemeinsam, dass sie nur zu einem Teilumsatz an HFP führen, das heisst, dass Gemische aus gebildetem HFPO und unumgesetztem HFP anfallen. Aus diesen Gemischen soll das unumgesetzte HFP vor der Weiterverarbeitung des HFPO möglichst quantitativ abgetrennt und in den Oxidationsprozess zurückgeführt werden. Diese Trennung ist jedoch schwierig.

Es ist bereits versucht worden, das in solchen Gemischen anwesende HFP durch Übeführung in sein Dibromid und anschliessende Destillation abzutrennen (DE-PS 12 40 516). Dies bedingt jedoch entweder den Verlust einer kostspielgen Substanz oder deren aufwendige Wiedergewinnung aus dem Dibromid.

Die destillative Reindarstellung von HFPO·aus Gemischen mit HFP ist technisch nicht einfach durchführbar, da die für eine Trennung bestimmenden physikalischen Eigenschaften der beiden Komponenten sehr ähnlich sind. Eine reine destillative Trennung würde aufgrund der eng beieinander liegenden Siedepunkte (HPF −29,4°C, HFPO −27,4°C) eine Destillationskolonne von über 80 m Länge erfordern.

Es hat daher nicht an Bemühungen gefehlt, die Flüchtigkeit von HFP durch Zusatz eines Lösungsmittels herabzusetzen und dadurch die relative Flüchtigkeit des HFPO zu erhöhen. In der DE-AS 12 51 300 wird ein Verfahren zur Reinigung von HFPO unter Abtrennung von HFP durch extraktive Destillation beschrieben, bei dem die Destillation in Gegenwart von alkyl- oder alkoxy-substituierten Benzolen, die mit Alkyl- oder Alkoxygruppen mit 1 bis 4 Kohlenstoffatomen mono-, di- oder trisubstituiert sind, von Dialkylethern von Ethylenglykol oder Diethylenglykol, wobei die Alkylgruppen der Ether 1 bis 2 Kohlenstoffatome aufweisen, oder von Tetrachlorkohlenstoff oder Chloroform durchgeführt wird. Ferner wird in der DE-OS 27 52 998 ein Verfahren zur Reinigung von HFPO unter Trennung des HFPO von HFP durch extraktive Destillation in Anwesenheit einer normalerweise flüssigen Verbindung zur Erhöhung der relativen Flüchtigkeit beschrieben, bei dem als normalerweise flüssige Verbindung ein chlorierter Kohlenwasserstoff mit mindestens 2 Kohlenstoffatomen oder ein Dialkylether mit mindestens einer Alkylgruppe, welche verzweigt ist und mindestens 3 Kohlenstoffatome aufweist, eingesetzt wird.

Durch Einsatz eines die relative Flüchtigkeit beeinflussenden Lösungsmittels (Extraktivmediums) kann die Grösse des zur Trennung von HFPO und HFP erforderlichen Apparates erheblich reduziert werden, was zu einer beträchtlichen Senkung der Kosten führt. Während die rein destillative Trennung eine Kolonnenlänge von über 80 m erfordern würde, kommt man bei Anwendung des Verfahrens der Extraktivdestillation mit einer Kolonnenlänge im Absorptionsteil von nur 5 bis 12 m aus. Dies entspricht einer theoretischen Bodenzahl von etwa 20 bis 50.

Die Ersparnis an Investitionskosten, die bei Einsatz des Verfahrens der extraktiven Destillation anstelle des Verfahrens der reinen Destillation resultiert, wird jedoch durch einen erhöhten Energieverbrauch zunichte gemacht, der zur Erhitzung und Kühlung des im Kreis geführten Extraktivmediums erforderlich wird, insbesondere dann, wenn das Extraktivmedium nicht mit Kühlwasser, sondern mit Kühlsole auf Temperaturen unter 15°C gekühlt wird, um die Gaslöslichkeit zu erhöhen.

Der vorliegenden Erfindung liegt die Aufgabe zugrunde, den für die Trennung von HFPO und HFP nach dem Verfahren der extraktiven Destillation erforderlichen Energiebedarf zu verringern, indem ein den bisher beschriebenen Verbindungen in seinem Trenneffekt überlegenes Extraktivmedium eingesetzt wird.

Diese Aufgabe wird gelöst durch ein Verfahren zur Abtrennung von HFPO aus Gasgemischen, bestehend aus HFPO und HFP, durch extraktive Destillation in Gegenwart eines Extraktivmediums, das dadurch gekennzeichnet ist, dass das Gasgemisch mit Methylenchlorid als Extraktivmedium in Berührung gebracht wird, wobei HFPO gasförmig entweicht und HFP absorbiert wird, das dann durch Erwärmen des Methylenchlorids auf seinen Siedepunkt wieder in Freiheit gesetzt wird.

Überraschenderweise wurde gefunden, dass Methylenchlorid über eine bessere Trennleistung verfügt und sich für die Trennung von HFPO und HFP besser eignet als die für diesen Zweck bereits beschriebenen Chlorkohlenwasserstoffe mit 1 oder 2 Kohlenstoffatomen, wie beispielsweise Chloroform, Tetrachlorkohlenstoff, 1,2-Dichlorethan oder Trichlorethylen.

Die relative Flüchtigkeit von HFP/HFPO, definiert als die Gaslöslichkeit von HFP, dividiert durch die Gaslöslichkeit von HFPO (gemessen in $cm^3$ pro g Lösungsmittel und bar bei 20°C) ist bei Einsatz von Methylenchlorid 20 bis 30° höher als bei Verwendung anderer homologer Chlorkohlenwasserstoffe. Dieser Sachverhalt geht aus folgender Tabelle hervor:

| Extraktivmedium | Relative Flüchtigkeit (HFP/HFPO) |
|---|---|
| Methylenchlorid | 2,14 |
| Chloroform | 1,77 |

| Extraktivmedium | Relative Flüchtigkeit (HFP/HFPO) |
|---|---|
| 1,2-Dichlorethan | 1,72 |
| Tetrachlorkohlenstoff | 1,67 |
| Trichlorethylen | 1,49 |

Da der Energieverbrauch für die Trennung von HFP und HFPO mit steigender relativer Flüchtigkeit streng proportional abnimmt, wird bei Einsatz von Methylenchlorid anstelle der bislang für die Trennung eingesetzten Chlorkohlenwasserstoffe eine 20 bis 30%ige Energieersparnis erzielt.

Daneben weist Methylenchlorid als Extraktivmedium für diese Trennung noch eine Reihe von Vorteilen auf, die in dieser Kombination den bekannten Extraktivmedien für diesen Prozess im allgemeinen nicht zukommen:

1. es bildet mit HFP und HFPO kein azeotropes Gemisch,
2. es liegt am Siedepunkt von HFP und HFPO noch flüssig vor,
3. es geht mit HFP und HFPO unter Betriebsbedingungen keinerlei chemische Reaktionen ein,
4. es besitzt bei Normaldruck den niedrigen Siedepunkt von 40°C, was gleichfalls Energieersparnis bedeutet,
5. es ist billig und leicht erhältlich,
6. es ist nicht brennbar,
7. es hält auch der wiederholten thermischen Beanspruchung unter Betriebsbedingungen stand,
8. es ist toxikologisch unbedenklich.

Das erfindungsgemässe Verfahren wird zweckmässigerweise in kontinuierlicher Arbeitsweise wie folgt durchgeführt: Man leitet das zu trennende Gasgemisch in den unteren Teil einer Absorptionskolonne mit 20 bis 50 theoretischen Böden ein. In dieser Absorptionskolonne, die beispielsweise als Füllkörperkolonne oder als Glokkenbodenkolonne ausgebildet sein kann, strömt flüssiges Methylenchlorid von oben nach unten. Am Kopf der Absorptionskolonne wird das im Methylenchlorid weniger lösliche HFPO in konzentrierter Form abgenommen, während sich HFP im Methylenchlorid löst. Das mit HFP beladene Methylenchlorid wird, von der Absorptionskolonne kommend, in eine Kolonne mit etwa 10 theoretischen Böden befördert, die mit einer Sumpfheizung ausgestattet ist. Das Methylenchlorid wird in dieser Kolonne zum Sieden erhitzt. Dabei entweicht das gelöste HFP und wird am Kopf dieser Kolonne abgenommen. Das entgaste Methylenchlorid wird sodann wieder auf die gewünschte Absorptionstemperatur abgekühlt und erneut dem Kopf der Absorptionskolonne zugeführt.

Der Absorptionsvorgang erfolgt zweckmässigerweise in einem Temperaturbereich von −20°C bis +30°C, vorzugsweise von +15°C bis +25°C. Absorption und Desorption können bei gleichem Druck erfolgen. Um in beiden Kolonnen den gleichen Druck einzustellen, kann der Kopf der Desorptionskolonne mit dem unteren Teil der Absorptionskolonne durch eine Druckausgleichsleitung verbunden werden.

Der angewandte Druck ist an sich nicht kritisch und kann im Bereich von 0,8 bis 7 bar (absolut) liegen. Die obere Druckgrenze für die Durchführung des Verfahrens ist so zu bemessen, dass der Verflüssigungsdruck für HFP und HFPO bei der herrschenden Arbeitstemperatur nicht erreicht wird.

Die umzuwälzende Menge an Methylenchlorid wird bei gegebener Zahl an theoretischen Böden im wesentlichen von der Absorptionstemperatur und der angestrebten Reinheit des HFPO- und HFP-Produktstroms bestimmt.

Zur Kühlung des im Kreis geführten Methylenchlorids wird zweckmässigerweise Kühlwasser als kostengünstigstes Kühlmedium eingesetzt. Bei Verwendung von Kühlwasser wird eine Absorptionstemperatur im Bereich von 15 bis 25°C erreicht. In diesem Temperaturbereich werden für das Trennverfahren 75 bis 125 kg Methylenchlorid pro kg zu trennendes HFPO/HFP-Gemisch benötigt.

Es hat sich gezeigt, dass Methylenchlorid unter den Arbeitsbedingungen des erfindungsgemässen Verfahrens im Dauerbetrieb absolut stabil ist und ohne Regeneration ein Jahr oder länger eingesetzt werden kann, ohne dass seine Trennwirkung in irgendeiner Weise beeinträchtigt wird. Im Dauerbetrieb ist Methylenchlorid den früher beschriebenen Ethern überlegen. Diese Ether sind als Extraktivmedium zur Trennung von HFPO/HFP-Gemischen auch deswegen weniger geeignet, da sie unter den Betriebsbedingungen chemisch langsam verändert werden. Gebildete Reaktionsprodukte müssen daher von Zeit zu Zeit ausgeschleust und durch neues Extraktivmedium ersetzt werden.

Die Erfindung wird in den folgenden Beispielen näher erläutert:

Beispiel 1

In eine Absorptionskolonne mit einem Innendurchmesser von 100 mm und einer Höhe von 7 m, gefüllt mit Raschig-Ringen mit einem Durchmesser von 10 mm, werden 7 kg/h eines Gasgemisches, bestehend aus 89 Gew.-% HFP und 11 Gew.-% HFPO eingeleitet. Die Einleitstelle befindet sich 3 m über dem unteren Ende der Absorptionskolonne. Am Kopf der Absorptionskolonne werden 662 kg/h Methylenchlorid aufgegeben, das mit Kühlwasser auf +22°C gekühlt wird. Der Druck in der Absorptionskolonne beträgt 1,7 bar (absolut). Der am Kopf der Absorptionskolonne entweichende HFPO-Produktstrom enthält noch partialdruckmässig mitgeführtes Methylenchlorid. Dieses Methylenchlorid wird über eine nachgeschaltete kleine Destillationskolonne, die mit einem Rücklaufverhältnis von 1:1 betrieben wird, abgeschieden. Der von Methylenchlorid freie HFPO-Produktstrom von 720 g/h enthält 98,6 Gew.-% HFPO und 1,4 Gew.-% HFP.

Das die Absorptionskolonne am unteren Ende verlassende, mit Hexafluorpropylen beladene Methylenchlorid wird in der Mitte einer 3 m langen Desorptionskolonne aufgegeben. Die Desorptionskolonne besitzt den gleichen Durchmesser und die gleichen Füllkörper wie die Absorptionskolonne. Der Kopf der Desorptionskolonne ist mit dem unteren Teil der Absorptionskolonne durch eine Druckausgleichsleitung verbunden. Die Desorptionskolonne ist mit einem Umlaufverdampfer versehen, in dem das Methylenchlorid zum Sieden erhitzt wird. Am Kopf der Desorptionskolonne befindet sich ein mit Kühlsole betriebener Kondensator, mit dem das im Umlaufverdampfer verdampfte Methylenchlorid und ein Teil des HFP wieder kondensiert wird. Über den Konensator werden 6280 g/h gasförmiges Produkt abgenommen, das 99 Gew.-% HFP und 1,0 Gew.-% HFPO enthält.

Beispiel 2

Gearbeitet wird genau wie in Beispiel 1, mit dem Unterschied, dass das der Absorptionskolonne zugeführte HFPO/HFP-Gemisch 62 Gew.-% HFPO und 38 Gew.-% HFP enthält. Das am Kopf der Absorptionskolonne in einer Menge von 4335 g/h abgenommene gasförmige Produkt enthält 99 Gew.-% HFPO und 1 Gew.-% HFP. Über Kopf der Desorptionskolonne werden 2665 g/h gasförmiges Produkt mit der Zusammensetzung 98,2 Gew.-% HFP und 1,8 Gew.-% HFPO entnommen.

Beispiel 3

Es wird analog Beispiel 2 verfahren, jedoch wird die im Kreis gefahrene Methylenchloridmenge von 662 kg/h auf 875 kg/h erhöht.

Der am Kopf der Absorptionskolonne mit 4338 g/h entnommene Produktstrom enthält 99,6 Gew.-% HFPO und 0,4 Gew.-% HFP. Am Kopf der Desorptionskolonne werden 2662 g/h eines Produktes der Zusammensetzung 99,3 Gew.-% HFP und 0,7 Gew.-% HFPO entnommen.

**Patentanspruch**

Verfahren zur Abtrennung von Hexafluorpropylenoxid aus Gasgemischen, bestehend aus Hexafluorpropylenoxid und Hexafluorpropylen, durch extraktive Destillation in Gegenwart eines Extraktivmediums, dadurch gekennzeichnet, dass das Gasgemisch mit Methylenchlorid als Extraktivmedium in Berührung gebracht wird, wobei Hexafluorpropylenoxid gasförmig entweicht und Hexafluorpropylen absorbiert wird, das dann durch Erwärmen des Methylenchlorids auf seinen Siedepunkt wieder in Freiheit gesetzt wird.

**Claim**

A process for the removal of hexafluoropropylene oxide from gas mixtures composed of hexafluoropropylene oxide and hexafluoropropylene by extractive distillation in the presence of an extractive medium characterised by bringing the gas mixture into contact with methylene chloride as the extractive medium whereupon hexafluoropropylene oxide is evolved in the form of gas and hexafluoropropylene is absorbed and is then liberated by heating the methylene chloride to its boiling point.

**Revendication**

Procédé pour séparer l'oxyde d'hexafluoropropène de mélanges gazeux constitués d'oxyde d'hexafluoropropène et d'hexafluoropropène, par distillation extractive en présence d'un milieu extractif, procédé caractérisé en ce qu'on met le mélange gazeux en contact avec du chlorure de méthylène comme milieu extractif, l'oxyde d'hexafluoropropène se dégageant alors à l'état gazeux et l'hexafluoropropène étant absorbé, puis ce denrier est remis en liberté par chauffage du chlorure de méthylène à son point d'ébullition.